# EUROPEAN PATENT SPECIFICATION

(11) **EP 1 597 351 B1**
(45) Date of publication and mention of the grant of the patent: **18.04.2012**
(21) Application number: 04714072.8
(22) Date of filing: 24.02.2004
(51) Int. Cl.: C12Q 1/68

(54) **METHODS AND CONSTRUCTS FOR EVALUATION OF RNAi TARGETS AND EFFECTOR MOLECULES**
VERFAHREN UND KONSTRUKTE ZUR BEWERTUNG VON RNAi-ZIELEN UND EFFEKTORMOLEKÜLEN
METHODES ET PRODUITS DE RECOMBINAISON POUR L'EVALUATION DE CIBLES D'ARNi ET MOLECULES EFFECTRICES

(30) Priority: 27.02.2003 US 451070 P
(43) Date of publication of application: 23.11.2005
(73) Proprietor: Alnylam Pharmaceuticals Inc., Cambridge MA 02142 (US)
(72) Inventor: PACHUK, Catherine, J., Lansdale, PA 19446 (US)
(74) Representative: HOFFMANN EITLE
(86) International application number: PCT/US2004/005065
(87) International publication number: WO 2004/076629

(56) References cited:
- WO-A2-02/44321
- US-A- 5 738 985
- US-A1- 2002 132 257
- US-A1- 2002 132 257
- US-A1- 2002 164 636
- CHOPRA M ET AL: "Using RNA interference to modulate gene expression" TARGETS, ELSEVIER, vol. 1, no. 3, 1 September 2002 (2002-09-01), pages 102-108, XP004886602 ISSN: 1477-3627
- KUMAR R ET AL: "High-throughput selection of effective RNAi probes for gene silencing" GENOME RESEARCH, COLD SPRING HARBOR LABORATORY PRESS, WOODBURY, NY, US, vol. 13, no. 10, October 2003 (2003-10), pages 2333-2340, XP002292586 ISSN: 1088-9051
- MEI HUANG ET AL: "Fast screening target sites for RNA interference using a cell-free system" BIOTECHNOLOGY LETTERS, vol. 27, no. 9, May 2005 (2005-05), pages 619-621, XP002377333 ISSN: 0141-5492
- LI YX ET AL: "Double-stranded RNA injection produces null phenotypes in zebrafish", DEVELOPMENTAL BIOLOGY, ACADEMIC PRESS, NEW YORK, NY, US, vol. 217, no. 2, 15 January 2000 (2000-01-15), pages 394-405, XP002138444, ISSN: 0012-1606, DOI: DOI:10.1006/DBIO.1999.9540

## Description

### FIELD OF THE INVENTION

This invention relates to the field of post-transcriptional gene silencing (PTGS) or RNA interference (RNAi), a mechanism widely found in plant and animals cells, which produces silencing or inhibition of a gene homologous to a double-stranded RNA (dsRNA) introduced into the cell. More particularly, this invention relates to methods and constructs for selecting dsRNAs and/or targets for utilization in dsRNA-mediated RNAi.

### BACKGROUND OF THE INVENTION

Double-stranded RNAs are known to trigger silencing of a target gene having a nucleotide sequence complementary to one strand of the double-stranded RNA structure, believed to involve degradation of the mRNA transcribed from the target gene. This phenomenon, termed post-transcriptional gene silencing (PTGS) or RNA interference (RNAi), is probably an evolutionarily conserved defense mechanism against viruses and the mobilization of transposons, and is found in plants, invertebrates including *C. elegans* and *Drosophila*, and in vertebrates, e.g., fish, mammals, including humans. RNAi promises broad applicability to reduce or eliminate the generation of abnormal and/or undesired gene products, including those from transgenes, endogenous genes, and pathogen genes. Introducing dsRNA into cells having the required molecular machinery triggers processing of the dsRNA into short segments which associate with cellular proteins to initiate degradation of homologous mRNAs. PTGS presents a new and exciting approach for down-regulating or silencing the expression of genes.

RNAs having a double-stranded sequence as short as about 19 nucleotides in length may be effective to produce gene silencing, but, in general, longer dsRNAs, e.g., several hundred to several thousand nts in length are more effective. While there is no real upper limit, maximum length is determined primarily as a matter of convenience and practicality, involving such matters as synthesis and delivery of the desired dsRNAs. Currently, however, there are no rules for selection of optimal targets and effectors for RNAi and there is a need for efficient methods for evaluating target sequences and potential dsRNA effector molecules.

### SUMMARY OF THE INVENTION

The assay method of the invention is defined in claims 1-16 and 23 and provides a rapid, efficient assay for evaluating potential mRNA targets for dsRNA-mediated silencing or degradation, as well as effector dsRNA molecules for utilization in such silencing mechanisms. The method utilizes a reporter-target sequence fusion message construct, comprising an mRNA encoding a reporter sequence linked to a target sequence. The target sequence is selected to determine its amenability to dsRNA-associated degradation. The reporter sequence and the target sequence to be evaluated are present within a capped and polyadenylated mRNA transcript capable of being translated within an appropriate cell line or other system. Translation of the mRNA will result in a production of a detectable reporter. The target sequence is present within the either the 5' or 3' untranslated region of the mRNA.

-The mRNA fusion construct is contacted with an RNA molecule(s) having a double-stranded portion complementary to at least a portion of the target sequence, under conditions in which dsRNA-associated degradation of the corresponding mRNA sequence can occur. If cleavage of the mRNA transcript does occur, translation of the reporter cannot occur and there will be a
US 2002/132257 A1 concerns the use of post-transcriptional gene silencing for identifying nucleic acid sequences that modulate the function of a cell. WO 02/44321 concerns RNA interference mediating small RNA molecules. Lie et al; Dev. Biol., vol. 217, 2000, pages 394-405 concerns double-stranded RNA injection resulting in null phenotypes in Zebrafish. US 6,013,447 concerns random intracellular method for obtaining optimally active nucleic acid molecules. detectable elimination, diminution, or modulation of the reporter gene product. EGFP is a particularly preferred reporter for use in such a screening assay because its modulation can be directly monitored *in situ*, without the need for tedious and time-consuming analytical steps, such as cell lysis, recovery of reporter, etc. Other GFP variants are also suitable, as are other reporters capable of convenient detection, particularly chemiluminescent, fluorometric, and colorimetric reporter systems.

The invention also provides a RNA, competent cell system as defined in claims 17-22 and 29.

### BRIEF DESCRIPTION OF FIGURES

FIGURE 1 is a depiction of the EGFP-fusion mRNA assay.

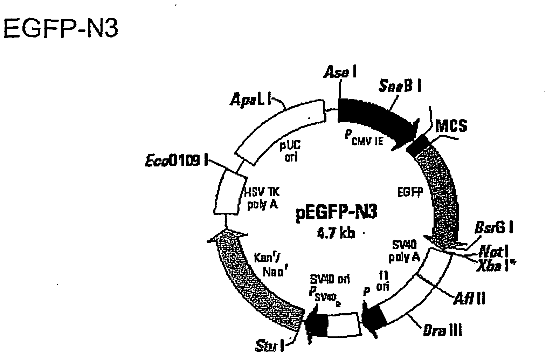

FIGURE 2 depicts plasmid pEGFP-N3, which encodes a red-shifted variant of wild-type GFP (1-3) which has been optimized for brighter fluorescence and higher expression in mammalian cells. (Excitation maximum = 488 nm; emission maximum = 507 nm.) pEGFP-N3 encodes the GFPmut1 variant (4) which contains the double-amino-acid substitution of Phe-64 to Leu and Ser-65 to Thr. The coding sequence of the EGFP gene contains more than 190 silent base changes which correspond to human codon-usage preferences (5). Sequences flanking EGFP have been converted to a Kozak consensus translation initiation site (6) to further increase the translation efficiency in eukaryotic cells. The MCS in pEGFP-N3 is between the immediate early promoter of CMV (P_{cmv IE}) and the EGFP coding sequences. Genes cloned into the MCS will be expressed as fusions to the N-terminus of EGFP if they are in the same reading frame as EGFP and there are no intervening stop codons. SV40 polyadenylation signals downstream of the EGFP gene direct proper processing of the 3' end of the EGFP mRNA. The vector backbone also contains an SV40 origin for replication in mammalian cells expressing the SV40 T.
FIGURE 3 depicts a fusion mRNA structure in upper left hand corner. The sense strand of siRNA#1 is represented in the HBVsAg target sequence. Fluorescent micrographs of transfections A, B, and C are shown.
FIGURE 4 shows HBVsAg levels as determined by ELISA for: (left panel) cells transfected with HBVsAg expression vector and siRNA#2, (middle panel) cells transfetced with HBVsAg expression vector and siRNA#1 and (right panel) cells transfected with HBVsAg and no siRNA. All measurements were made at 18hrs post-transfection. Duplicate measurements for each experiment are shown.

### DETAILED DESCRIPTION OF THE INVENTION

The assay method of the invention provides a rapid, efficient assay for evaluating potential mRNA targets for dsRNA-mediated silencing or degradation, as well as effector dsRNA molecules for utilization in such silencing mechanisms. Although there has been considerable discussion and debate about the relative merits of short dsRNAs (siRNAs) vs. long dsRNAs, exogenously introduced vs. endogenously expressed dsRNAs, there has been little consideration of other factors that may be important in selection of gene targets particularly amenable to RNAi, or, on the other hand, selection of particularly effective dsRNAs for use in dsRNA-mediated gene silencing. Accordingly, there is a great need for rapid, efficient assay methods designed to enable evaluation of target sequences as well as dsRNA effector molecules for use in PTSG.

As to factors of importance in the efficiency of PTGS, the RNA sequence of the dsRNA effector molecule selected and of its corresponding mRNA target are expected to significantly impact the efficiency of PTGS. It has been shown that not all potential target sequences will be equally amenable to silencing. Similarly, not all dsRNA effector molecules will be equally efficient in producing the desired effect. In part this is due to the variable three-dimensional structure associated with different RNA sequences. Since RNAs are known to fold according to nearest neighbor rules, they assume various secondary structures as they are synthesized. The particular three-dimensional structure formed and the strength of the bonds holding an RNA molecule in a particular conformation will be dependent upon the sequence of the molecule. Protein-binding to various regions of RNAs and other currently unrecognized factors may also be relevant. A particular RNA strand may therefore vary greatly in its availability to hybridize with a complementary oligonucleotide strand to form a double-stranded structure, or for inverted repeat sequences within a single oligonucleotide strand to form a stem-loop structure. This is particularly true when it is desired to transcribe two complementary RNAs from one or more expression vector(s) with the intent that they hydridize to form a dsRNA, or to express a single RNA strand with inverted repeats or self-complementary regions, capable of forming a stem-loop or hairpin dsRNA structure. This obstacle to forming dsRNAs may also be encountered when complementary RNA strands are synthesized *in vitro* and brought together under conditions designed to permit hybridization into dsRNAs. In addition, the different siRNA molecules which are processed from the larger input dsRNA molecules may interact with different affinities with proteins/complexes involved in gene silencing, influencing their ability to achieve silencing.

The screening assay of the invention utilizes a reporter-target fusion message construct, comprising an mRNA encoding a reporter sequence linked to a target sequence. The target sequence is selected to determine its amenability to dsRNA-associated degradation. The reporter sequence and the target sequence to be evaluated are present within a capped and polyadenylated mRNA transcript capable of being translated within an appropriate cell line or other system. Accordingly, the functional mRNA will comprise either a 5' cap or an IRES element, a suitable 5' untranslated region (UTR), a coding sequence for a selected reporter, a PTGS target sequence, a 3' untranslated region, and a poly (A) tail at the 3' end. The 5' UTR will contain the regulatory elements required for translation in the selected assay system, e.g., a Kozak sequence flanking the translation start codon. Translation of the mRNA will result in production of a detectable reporter. The target sequence may conveniently be placed within the 5' UTR in a position which does not interfere with initiation of translation, or within the 3' UTR. In a preferred embodiment, the target sequence is present after the translation stop codon of the reporter sequence, within the 3' untranslated region of the mRNA.

The mRNA fusion construct is contacted with an RNA molecule(s) having a double-stranded portion complementary to at least a portion of the target sequence, under conditions permitting dsRNA-associated degradation of the corresponding mRNA sequence. If there is cleavage of the mRNA transcript anywhere between the cap and the polyA tail, translation of the reporter cannot occur and there will be a detectable elimination, diminution, or modulation of the reporter gene product. A chemiluminescent or fluorometric reporter will be advantageous in the methods of the invention. EGFP is a particularly preferred reporter for use in such a screening assay because its modulation can be directly monitored real time and *in situ*, obviating the need for tedious and time-consuming analytical steps, such as cell lysis, recovery of reporter, etc. Other GFP variants are also suitable, as are other reporters capable of convenient detection.

The target sequence can represent virtually any target (or targets) including without limitation of prokaryotic, eukaryotic, plant, animal, invertebrate, vertebrate origin, selected for potential dsRNA-mediated down regulation, e.g., any pathogen of plant or animal, e.g., fungal, bacterial, viral, or prion pathogen sequence(s), e.g., a sequence from HIV, HSV, HBV, HCV, HPV, smallpox, anthrax, etc.; an endogenous gene associated with pathology or disease, such as TNF, a cancer-associated gene, or a host gene responsible for entry or infection by a pathogen; or a transgene, e.g., a gene introduced for gene therapy purposes, to be modulated or down-regulated. Among cancer-associated genes are included cancers of any type, in any species, e.g., developmental genes, cytokines/lymphokines and their receptors, growth/differentiation factors and their receptors, neurotransmitters and their receptors, oncogenes, the BCR-abl chromosomal sequences, tumor suppressor genes, enzymes, etc.(see the teaching of e.g., U.S. 6,506,559 B1). Among viral genes selected for evaluation using the method of the invention are included, without limitation, viruses of the species *Retrovirus*, *Herpesvirus*, *Hepadnavirus*, *Poxvirus*, *Parvovirus*, *Papillornavirus*, and *Papovavirus*. Specifically, some of the more desirable viruses to evaluate with this method include, without limitation, HIV, HBV, HCV, HSV, CMV, HPV, HTLV and EBV. In particular, a viral polynucleotide sequence necessary for replication and/or pathogenesis of the virus in an infected mammalian cell is selected. Among such target polynucleotide sequences are protein-encoding sequences for proteins necessary for the propagation of the virus, e.g., the HIV gag, env and pol genes; the HPV6 L1 and E2 genes; the HPV11 L1 and E2genes; the HPV16 E6 and E7 genes; the HPV18 E6 and E7 genes; the HBV surface antigens, the HBV core antigen, HBV reverse transcriptase; the HSV gD gene, the HSV vp16 gene, the HSV gC, gH, gL and gB genes, the HSV ICP0, ICP4 and ICP6 genes; Varicella zoster gB, gC and gH genes; and non-coding viral polynucleotide sequences which provide regulatory functions necessary for transfer of the infection from cell to cell, e.g., the HIV LTR, and other viral promoter sequences, such as HSV vp16 promoter, HSV-ICP0 promoter, HSV- ICP4, ICP6 and gD promoters, the HBV surface antigen promoter, the HBV pre-genomic promoter, among others.

The target sequence can be any heterologous sequence from about 19 nucleotides in length, up to about 8,000 nts, and may advantageously include sequences representing two or more epitopes from a single target, i.e., a single gene of interest, or different genes from the same organism, or one or more sequences from a number of different targets, such as different viruses, e.g., HIV, HBV, HCV, smallpox, etc.

The fusion mRNAs of the invention can be transiently or stably expressed in cells capable of carrying out PTGS. Alternatively, the fusion mRNAs can be transcribed *in vitro* and introduced into such cells by any of a number of known delivery mechanisms, such as injection, electroporation, transfection with one or more of the many known RNA or DNA delivery agents (also suitable for delivery of plasmid expression vectors expressing fusion mRNAs), (e.g., Lipofectamine^{™}; Fugene^{™}; cationic lipids; cationic amphiphiles; local anesthetics such as bupivacaine, as in U.S. 6,217,900; complexes comprising a cationic polyamine and an endosome disruption agent, as in U.S. 5,837,533 and 6,127,170; calcium phosphate, etc.).

Similarly, the effector dsRNAs can be synthesized using known methods or they can be transcribed and assembled *in vitro* and introduced by similar means into the test cells or expressed within such cells from a vector(s) (e.g., DNA plasmid or viral vector). The effector dsRNAs can be short (with a double-stranded region of at least about 19 nts) or long (50, 100, 200, e.g., several hundred to several thousand nts), comprised of separate complementary single strands, or of a single strand with inverted complementary regions and optionally a spacer region which will form a stem-loop or hairpin structure with a double-stranded region or regions (of at least 19 nts, or longer regions of 50, 100, several hundred to several thousand nts) complementary to at least part of the target sequence in the fusion mRNA to be evaluated. The effector dsRNAs can advantageously be any at least partially double-stranded RNA molecule having a double-stranded region of at least about 19 nucleotides homologous to a target sequence and otherwise capable of mediating RNAi, including RNA/DNA duplexes, circular RNAs with self-complementary regions that hybridize to form a partially-double-stranded structure, lariat structures, single-stranded hairpin structures, double-stranded structures, etc., described in detail, including synthetic methods, in WO 0063364 A2, "Methods and Compositions for Inhibiting the Function of Polynucleotide Sequences", C. Pachuk, and C. Satishchandran; still other dsRNA effector molecules desirable for use in the methods of this invention and methods for making them are described in U.S. Provisional Application 60/399,998, filed 31-Jul-2002.

The dsRNA effector molecules to be evaluated may be made *in vitro* by conventional enzymatic synthetic methods using, for example, the bacteriophage T7, T3 or SP6 RNA polymerases according to the conventional methods described by such texts as the Promega Protocols and Applications Guide, (3rd ed. 1996), eds. Doyle, ISBN No. 1-882274-57-1. See also: http://www.promega.com/guides. Alternatively, the shorter dsRNA molecules (e.g., less than about 300 nts) may be made by chemical synthetic methods *in vitro* [see, e.g., Q. Xu et al, Nucl. Acids Res., 24(18):3643-4 (Sept. 1996); N. Naryshkin et al., Bioorg. Khim., 22(9): 691-8 (Sept. 1996); J. A. Grasby et al, Nucl. Acids Res., 21(19):4444-50 (Sept 1993); C. Chaix et al., Nucl. Acids Res., 17(18):7381-93 (1989); S. H. Chou et al., Biochem. 28(6):2422-35 (Mar. 1989); O. Odal et al., Nucl. Acids Symp.Ser., 21:105-6(1989); N. A. Naryshkin et al., Bioorg. Khim, 22(9):691-8 (Sept. 1996); S. Sun et al, RNA, 3(11):1352-1363 (Nov. 1997); X. Zhang et al, Nucl. Acids Res., 25(20), 3980-3 (Oct. 1997); S. M. Grvaznov et al., Nucl. Acids Res., 26 (18):4160-7 (Sept. 1998); M. Kadokura et al, Nucl. Acids Symp. Ser, 37:77-8 (1997); A. Davison et al, Biomed. Pept. Proteins. Nucl. Acids, 2(I):1-6(1996); and A.V. Mudrakovskaia et al., Bioorg. Khim., 17(6):819-22 (Jun. 1991)]. In addition, short dsRNAs are commercially available from sources including Dharmacon, Lafayette, CO.

The effector dsRNA molecules of this invention can also be made in a recombinant microorganism, e.g., bacteria and yeast or in a recombinant host cell, e.g., mammalian cells, and isolated from the cultures thereof by conventional techniques. *See*, e.g., the techniques described in Sambrook et al, Molecular Cloning: A Laboratory Manual, 3rd Ed.; Cold Spring Harbor Laboratory Press, Cold Spring Harbor, New York, 2000, which is exemplary of laboratory manuals that detail these techniques, and the techniques described in U.S. Pat. Nos. 5,824,538; 5,877,159; 5,643,771, and US Published Application 20020132257 A1.

Alternatively, the dsRNA effector molecules to be evaluated in the present invention may be co-expressed together with the reporter-target fusion message *in vivo* within the same cell in which the assay is carried out. Any suitable vector(s), the same or different, known to those of skill in the art may be used to express the dsRNA effector molecule, and/or the reporter-target fusion message, including a DNA single-stranded or double-stranded plasmid or vector. In a preferred embodiment, the agent which delivers the dsRNA effector and/or the reporter-target fusion message is a double-stranded DNA plasmid "encoding" the desired RNA molecule(s). See, e.g., the teaching of Sambrook et al., Molecular Cloning: A Laboratory Manual, 3rd Ed.; Cold Spring Harbor Laboratory Press, Cold Spring Harbor, New York, 2000. The fusion message RNAs are designed to be capped, and, if desired, cytoplasmic capping may be accomplished by various means including use of a capping enzyme such as a vaccinia capping enzyme or an alphavirus capping enzyme. The DNA vector is designed to contain one of the promoters or multiple promoters in combination (mitochondrial, RNA poll, II, or polIII, or viral, bacterial or bacteriophage promoters along with the cognate polymerases). Such plasmids or vectors can include plasmid sequences from bacteria, viruses, or phages. Such vectors include chromosomal, episomal and virus-derived vectors, e.g., vectors derived from bacterial plasmids, bacteriophages, yeast episomes, yeast chromosomal elements, and viruses, vectors derived from combinations thereof, such as those derived from plasmid and bacteriophage genetic elements, cosmids and phagemids. Thus, one exemplary vector is a single or double-stranded phage vector. Another exemplary vector is a single or double-stranded RNA or DNA viral vector. Such vectors may be introduced into cells as polynucleotides, preferably DNA, by well known techniques for introducing DNA and RNA into cells. The vectors, in the case of phage and viral vectors may also be and preferably are introduced into cells as packaged or encapsidated virus by well known techniques for infection and transduction. Viral vectors may be replication competent or replication defective. In the latter case, viral propagation generally occurs only in complementing host cells. In another embodiment the delivery agent comprises more than a single DNA or RNA plasmid or vector. As one example, a first DNA plasmid can provide a single-stranded RNA sense polynucleotide sequence as described above, and a second DNA plasmid can provide a single-stranded RNA antisense polynucleotide sequence as described above, wherein the sense and antisense RNA sequences have the ability to base-pair and become double-stranded. Such plasmid(s) can comprise other conventional plasmid sequences, e.g., bacterial sequences such as the well-known sequences used to construct plasmids and vectors for recombinant expression of a protein. However, it is desirable that the sequences which enable protein expression, e.g., Kozak regions, etc., are included in these plasmid structures only for expression of the reporter-target fusion message but not for expression of the dsRNA RNAi effector molecules.

### Screenin Assay

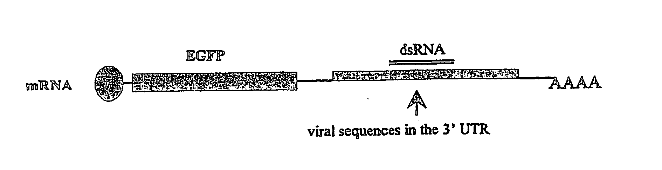

The inventors have developed a high throughput screening assay which enables the rapid screening of both target RNA sequences and effector dsRNA molecules. The basis of this assay is the use of a fusion mRNA, comprising a sequence encoding a reporter moiety linked to a sequence to be evaluated as a potential target for dsRNA-mediated gene silencing. In a preferred embodiment, utilization of an EGFP (Enhanced green fluorescent protein) fusion mRNA permits monitoring the "real-time" loss of expression of a targeted mRNA. The structure of such a fusion mRNA is depicted in Figure 1. Briefly, the fusion mRNA expresses EGFP due to the location of the EGFP coding region at the 5'end of the mRNA. The 3'UTR (untranslated region) includes a variable region in which different target sequences will be cloned. These target sequences can be derived from endogenous genes, transgenes, pathogen genes, etc., or any sequence desired to be evaluated as a target for dsRNA-mediated degradation. Induction of PTGS directed against the target sequences will result in cleavage and thus non-translatability of the fusion mRNA: EGFP expression will be lost. We have demonstrated the ability to include target sequences as large as 3.5 Kb in the 3'UTR and thus large regions of viral genomes or other sequences can initially be assayed for their ability to be targeted by RNAi.

Target RNA screen: It has been demonstrated that not all regions of a target mRNA can be successfully targeted for PTGS (). Presumably this has to do with inaccessibility of certain regions of mRNA molecules to gene silencing machinery and is likely caused by protein binding to the RNA and/or structure of the RNA. It is therefore important to be able to screen for mRNAs that can be efficiently targeted for PTGS. Constructs expressing the reporter-target fusion mRNAs of the invention can be co-transfected individually with constructs that express effector dsRNA molecules (*see* Effector RNA Screening below). Alternatively, stable cell lines expressing the fusion mRNA can be created and these cell lines can be transfected with vectors expressing effector dsRNAs, or the effector dsRNAs can be prepared *in vitro* or in another cell line, and delivered through known means to such cell lines. The time course and magnitude of silencing will be monitored through EGFP expression.

Effector dsRNA Screening: Prior work has demonstrated that not all dsRNAs are equally efficient in degrading target mRNAs. Some are much more efficient at inducing silencing and the onset of silencing can vary from molecule to molecule. Although the rules involved are not defined, structure of the target mRNA and the dsRNA species are believed to play a role. The EGFP screening system of the invention can be utilized to identify an efficient and rapid acting dsRNA(s) against each desired target.

It will be recognized that the EGFP reporter gene and the EGFP-N3 vector present certain advantages for practicing the methods of the invention, but that any vector than includes a detectable reporter can be utilized as described herein. Chemiluminescent, fluorometric, and colorimetric reporter systems are especially convenient for use in the assay methods of the invention, including, e.g., a luminescent □-galactosidase reporter system, EGFP and Luciferase reporter systems, (Clontech); the FluorAce beta-glucuronidase Reporter Kit Assay (Bio-Rad); Phospha-Light™ Secreted Alkaline Phosphatase Reporter Gene Assay System (Applied Biosystems). Many other known reporters or drug resistance genes could readily be adapted to use in the described assay, including acetohydroxyacid synthetase (AHAS), alkaline phosphatase (AP), secreted alkaline phosphatase (SEAP), beta galactosidase (LacZ), beta glucuronidase (GUS), chloramphenicol acetyltransferase (CAT), horseradish peroxidase (HRP), luciferase (Luc), nopaline synthetase (NOS), octopine synthetase (OCS), as well as a variety of selectable markers that confer resistance to antibiotics such as ampicillin, chloramphenicol, gentamicin, hygromycin, kanamycin, lincomycin, methotrexate, phosphinothricin, puromycin, and tetracycline. In addition, any gene product which can be detected can serve as the reporter, since dsRNA-induced cleavage of the mRNA construct will result in detectably modulated or decreased production of the gene product.

Cells or cell lines useful for carrying out the methods of the invention must be capable of supporting dsRNA-mediated RNAi; in general, however, most cells or cell lines have this capability. In addition to cellular machinery for carrying out RNAi, the methods of the invention require a system capable of supporting translation of the mRNA fusion construct, and, in those cases where it is desired to express the dsRNAs rather than providing exogenously formed dsRNAs, also the capacity to transcribe the effector dsRNA molecules. It is convenient to carry out the methods in a readily available and well characterized cell line such as Human RD (rhabdomyosarcoma), HuH7, HeLa, NIH3T3, and HepG2; however, most cell lines are RNAi competent, including a great variety of cell lines available from, e.g., ATCC (American Type Culture Collection - see ATCC.org). In addition, primary cells isolated from a tissue or an organism can be utilized. In general, it may be desirable to utilize cells, such as RD cells or Huh7 cells, capable of exhibiting a dsRNA-mediated stress response, particularly when the dsRNA effector molecules, e.g., long exogenously introduced dsRNAs, tend to induce such responses. This is less important when using dsRNA effectors such as expressed long dsRNAs, which do not induce a dsRNA-mediated stress response. Some cell lines, such as HeLa cells, which are capable of supporting RNAi, but are not competent with respect to exhibiting dsRNA-induced stress responses, may be preferred in some instances.

While the utilization of a chemiluminescent or fluorometric reporter makes it highly efficient to carry out the methods of the invention with *in situ* or real-time monitoring in various cell lines, if desired, the assay can also be carried out in a cell lysate. Additionally a cell-free system utilizing an *in vitro* transcription-translation kit (e.g., TNT Quick Coupled Transcription/Translation System, Promega, Madison, WI) can also be used, together with a Dicer or Dicer-type protein that cleaves longer dsRNAs into siRNAs, e.g., the Dicer siRNA Generation Kit available commercially from Gene Therapy Systems, Inc. (see genetherapysystems.com/catalog).

### EXAMPLES

### Example 1:

### Construction of an mRNA Fusion Vector

Vector preparation: pEGFP-N3 (Figure 2) a commercially available vector obtained from Clontech [(BD Biosciences Clontech, 1020 East Meadow Circle, Palo Alto, CA 94303) GenBank Accession #: U57609, Clontech Catalog #6080-1, See Catalog PR 08395, published 30 August 2000, which provides a restriction map and detailed information about the vector, including the following: pEGFP-N3 encodes a red-shifted variant of wild-type GFP, which has been optimized for brighter fluorescence and higher expression in mammalian cells. pEGFP-N3 encodes the GFPmut1 variant which contains the double amino acid substitution of Phe-64 to Leu and Ser-65 to Thr. The coding sequence of the EGFP gene contains more than 190 silent base changes which correspond to human codon-usage preferences. Sequences flanking EGFP have been converted to Kozak consensus translation initiation site to further increase the translation efficiency in eukaryotic cells.

EGFP-N3 vector was restricted with Not I which cleaves after the EGFP stop codon. Following Not I digestion, the ends of the vector were blunted according to standard techniques (See, Molecular Cloning: A Laboratory Manual, Cold Spring Harbor Laboratory Press, 3rd Ed, December 2000. Eds., Sambrook et al.)

HBV target sequence: The HBV derived target sequence is derived from HBV strain G2.27246, GenBank Accession # AF090839 and maps from coordinates 1849 to 2888 of this sequence.

Cloning of target HBV sequence into pEGFP-N3: A blunt-ended DNA fragment comprised of the HBV target sequence, was ligated into the vector pEGFP-N3 prepared as described above. The resultant construct EGFP/HBVsAg is pEGFP-N3 with the HBV target sequence encoded in the 3'UTR of the EGFP mRNA. Note that the Not 1 site, into which the HBV target sequence was cloned in downstream from the EGFP stop codon but upstream from the polyadenylation site (Figure 2).

### EXAMPLE 1A

### Human RD Cells, Transfections and Summary of Results

Human RD cells (Rhabdomyosarcoma/*Human Embryonal Rhabdomyososarcoma*) (available from ATCC, as well as other sources) were co-transfected with:
A) EGFP/HBVsAg (Enhanced green fluorescent protein/Hepatitis B virus surface Ag) fusion mRNA construct and an siRNA derived from HBV (siRNA#1)[ note that siRNA#1 maps to a subset of the HBV derived sequences cloned into the 3'UTR of EGFP/HBVsAg];
B) EGFP/HBVsAg fusion mRNA construct and a control siRNA (siRNA#2); or C) EGFP-N3 (EGFP plasmid without HBVsAg sequences) and siRNA#1. EGFP expression was monitored by fluorescent microscopy for 7 days. EGFP expression was down-regulated from 2-7 days post-transfection only in those cells co- transfected with the EGFP/HBVsAg fusion mRNA construct and siRNA#1. Levels of fluorescence were not down-regulated in cells transfected with EGFP-N3 plus siRNA#1, EGFP/HBVsAg fusion mRNA construct plus siRNA#2, EGFP-N3 plasmid alone (data not shown), or EGFP/HBVsAg fusion mRNA construct alone (data not shown). This demonstrates that the selected HBVsAg sequence inserted into the mRNA fusion construct constitutes a suitable target sequence for dsRNA-mediated gene silencing. By constructing a vector expressing an mRNA comprising the HBsAg target sequence alone, without the reporter sequence, and carrying out an analogous experiment with the same siRNA and other dsRNAs comprising a sequence complementary to the HBVsAg target, it was demonstrated (See Example 1B, below) that cleavage of the HBVsAg mRNA will also occur when presented in a more native conformation (not as a fusion mRNA), and that translation and generation of the protein product will be prevented or decreased.

### Reagents:

| HBV siRNA sequence (siRNA#1): maps to coordinates 2172-2196 of GenBank Accession # AF090839 | |
|---|---|
| Top strand: | 5'ccuccaaucacucaccaaccuccug3' |
| Bottom strand: | 3'ggagguuagugagugguuggaggac5 |
| | |
| Control siRNA sequence (siRNA#2): not derived from HBV sequences | |
| Top Strand: | 5' agcuucauaaggcgcaugcuu3' |
| Bottom Strand: | 3-uuucgaaguauuccgcguacg5' |

| | |
|---|---|
| Note: siRNAs were chemically synthesized by Dharmacon (Lafayette, Colorado). Top strand and bottom strand of each siRNA set were annealed using standard techniques. Alternatively, siRNAs can also be prepared enzymatically using commercially available siRNA transcription kits such as the one available from Ambion. | |

### HBVsAg Fusion Vectors and siRNAs were constructed as described in Figure 1 above

### Human RD Cells, Transfections and Summary of Results:

Human RD cells were seeded into six-well plates such that they were between 80-90% confluency at the time of transfection. All transfections were performed using Lipofectamine (InVitrogen, Carlsbad, CA) according to manufacturer's directions. Nucleic acid concentrations were held constant at 4.3 ug for each transfection. The following nucleic acids were transfected in the indicated transfections:
Transfection A) 300ng EGFP/HBVsAg, 2ug siRNA#1 and 2ug pGL3-basic vector (an inert DNA plasmid used as filler DNA for transfection);
Transfection B) 300ng EGFP/HBVsAg, 2ug siRNA #2 and 2ug pGL3-basic;
Transfection C) 300ng EGFP-N3 and 2ug siRNA#1.

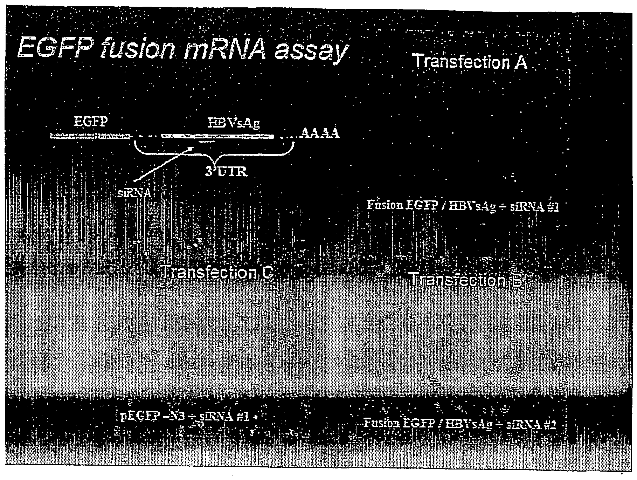

Transfection mixes were made using Lipofectamine and Opti-Mem (a serum-free medium available from InVitrogen), according to manufacturer's directions. The day after transfection, transfection mixes were removed from cells and replaced with DMEM (containing 10% FBS). This was designated one-day post-transfection. At days two-seven post-transfection, cells were visualized daily by both phase contrast microscopy and fluorescent microscopy. Cells belonging to the Transfection A group were significantly down-regulated for EGFP expression whereas cells belonging to Transfection B and Transfection C groups were not (Figure 3). No significant differences were observed in EGFP expression amongst not only the B and C transfection groups but also no differences were seen when B and C were compared to the fluorescence seen when cells were transfected in the same manner with the EGFP/HBVsAg fusion vector alone and/or the EGFP-N3 vector alone (data not shown). These results demonstrate that the EGFP/HBVsAg mRNA is specifically targeted by RNAi in cells transfected with the HBV siRNA#1, but not in cells transfected with the irrelevant siRNA#2. Also, as expected, the parental vector, PEGFP-N3, which does not contain any HBV sequences gave rise to an mRNA that was not targeted by either of the siRNAs. This experiment indicated that the selected HBV sequence utilized in the EGFP/HBVsAg construct is amenable to being targeted by RNAi and that siRNA#1 can effect RNAi of this target sequence.

### Example 1B.

To demonstrate that siRNA#1 can also target HBV sequences in a more native conformation, i.e., in the absence of EGFP mRNA sequences, the following experiment was done. An HBVsAg expression vector was constructed. This vector contains HBVsAg sequences derived from the HBV target sequence contained in the EGFP/HBVsAg fusion vector including those sequences corresponding to siRNA#1. The construct is designed to express middle sAg. Expression is directed by the HCMV promoter and the SV40 polyadenylation signal. Construction of such a vector can be easily accomplished by one skilled in the art.

In this experiment, RD cells were transfected with:
A) the HBVsAGg expression vector and siRNA#1;
B) the HBVsAg expression vector and siRNA#2; and
C) the HBVsAg expression vector alone.

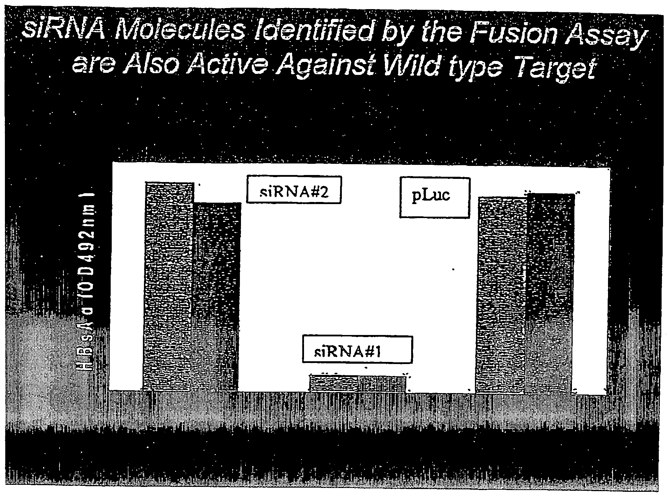

All transfections were performed as described for the fusion mRNA vector transfections using Lipofectamine. Transfection A contained 300ng HBVsAg expression vector, 2ug siRNA#1 and 2ug pGL3-basic vector; Transfection B contained 300ng HBVsAg expression vector, 2ug siRNA#2 and 2ug pGL3-basic vector; and Transfection C contained 300 ng HBVsAg expression vector and 2ug pGL3-basic vector. At 18 hrs post-transfection, media was collected from transfected cells and assayed for the presence of HBVsAg by ELISA. ELISA kits for the detection of HBVsAg are commercially available through Abbott Labs in Chicago. The results are shown in Figure 4. Briefly, siRNA#1 but not siRNA#2 was able to downregulate HBVsAg expression. Control levels of HBVsAg expression vector generated in transfections not containing any added siRNA is also shown.

### EXAMPLE 2

### Evaluation of Effector Molecules

After a suitable target region for PTGS has been identified, the assay of the invention can be utilized to evaluate the relative effectiveness of selected effector molecules which include a region of dsRNA complementary to the target mRNA. For example, a series of overlapping sequences complementary to a region of the HBVsAg in the EGFP-HBVsAg fusion mRNA construct described above is mapped out. The double-stranded region of such dsRNAs can be as short as 19 nts in length or as long as several thousand nts, but will preferably be 100, 200, up to 500 nts in length. The HBV derived target sequence utilized in the EGFP/HBVsAg Fusion Construct described above maps from coordinates 1849 to 2888 of the HBV strain G2.27246, GenBank Accession # AF090839, and thus represents a sequence of 1039 nts. Thus, e.g., the HVB derived target sequence can be divided up into overlapping stretches of approximately 200 nts, e.g., coordinates 1849-2050 (A), 1949-2150 (B), 2049-2250 (C); 2149-2349 (C); 2249-2450 (D); 2349-2550 (E); 2449-2649 (F); 2549-2749 (G); and 2649-2888 (H). Double stranded RNAs having one strand complementary and one strand homologous to this sequence are then synthesized, or, alternatively, are expressed as described herein, either as two separate strands synthesized individually and annealed under appropriate conditions, or as a single RNA strand having one such sequence in the sense orientation and another in the antisense orientation, preferably with a suitable linking region, e.g., a linking region of suitable length, e.g., 9 to 30 nucleotides, preferably consisting of a sequence of the same base, such as poly C, poly A, poly U, or poly G.

Human RD cells are transfected as described in Example 1 above with the EGFP/HBVsAg fusion vector and one of the dsRNA effector molecules to be evaluated, e.g., dsRNA A, dsRNA B, dsRNA C, dsRNA D, dsRNA E, dsRNA F, dsRNA G, or dsRNA H, or, alternatively, transcribed as described in Example 1 above with the EGFP/HBVsAg fusion vector and a vector designed to express one of the dsRNA effector molecules to be evaluated, e.g., dsRNA A, dsRNA B, ds RNA C, dsRNA D, dsRNA E, dsRNA F, dsRNA G, or dsRNA H. In each case, cells are visualized daily as described in Example 1A by both phase contrast microscopy and fluorescent microscopy, to determine the efficacy of each such dsRNA in eliciting RNAi and the relative efficacy of the eight dsRNAs.

### Example 3

In a further experiment, dsRNA effector molecules representing overlapping sequences of variable length, e.g., 20 nts, 30 nts, 50 nts, 100 nts, 150 nts, 200 nts, 300 nts, 400 nts, to 500 nts, mapping to the HBVsAg target sequence of the EGFP/HBVsAg Fusion Construct are designed and constructed, and delivered as described in Example 1 above, either as a synthesized dsRNA or as a vector expressing such a dsRNA, to appropriate cells such as Human RD or Huh7 cells, together with the EGFP/HSVsAg Fusion Construct. In each case, cells are visualized daily as described by both phase contrast microscopy and fluorescent microscopy, to determine the efficacy of each such dsRNA in eliciting RNAi and the relative efficacy of the various dsRNAs.

## Claims

1. A method for evaluating dsRNA-mediated silencing or inhibition of a target nucleotide sequence by a selected dsRNA effector molecule in an RNAi-competent system, comprising the steps of:
a) introducing into such system:
i) a capped and polyadenylated fusion mRNA encoding both a reporter gene sequence capable of translation in said system and a heterologous sequence to be evaluated as a target for RNAi (RNAi target sequence), wherein the RNAi target sequence is positioned within either the 5' or 3' untranslated region of the fusion mRNA; and
ii) a dsRNA effector molecule having an at least partially double-stranded RNA sequence, one strand of said sequence being substantially homologous to at least a portion of the RNAi target sequence; and
b) detecting the presence of the reporter gene product.

2. The method of claim 1 wherein the RNAi target sequence is positioned within the 3' untranslated region of the fusion mRNA.

3. The method of any of claims 1 or 2 wherein the reporter gene sequence encodes a chemiluminescent or fluorometric reporter.

4. The method of any of claims 1 or 3 wherein the reporter gene sequence encodes a fluorometric reporter.

5. The method of claim 4 wherein the fluorometric reporter is a green fluorescent protein (GFP).

6. The method of claim 5 wherein the reporter is EGFP.

7. The method of any of claims 1, or 3-6 wherein the RNAi target sequence is a sequence from a pathogen, an endogenous sequence associated with disease or pathology in a vertrebrate, or a transgene desired to be modulated.

8. The method of claim 7 wherein the pathogen is a virus, bacterium, fungus, nematode or a prion.

9. The method of claim 8 wherein the virus is HBV, HCV, HIV, HSV, HPV, CMV, EBV, or HTLV.

10. The method of claim 7 wherein the endogenous sequence is from TNF alpha, a cancer-associated sequence, or a host gene responsible for entry or infection by a pathogen.

11. The method of any of claims 1, or 2-10 in which the RNAi-competent system is a cell.

12. The method of claim 11 in which the cell is an RD cell, a Huh7 cell, or a HeLa cell.

13. The method of any of claims 1, or 2-12 in which the fusion mRNA is expressed within the cell.

14. The method of any of claims 1, or 2-13 in which the fusion mRNA is expressed from a plasmid.

15. The method of any of claims 1, or 2-14 in which the fusion mRNA and the effector dsRNA are both expressed within the cell.

16. The method of any of claims 1, or 2-15 in which both the fusion mRNA and the effector dsRNA are expressed from one or more plasmids.

17. A RNAi competent cell system comprising a capped and polyadenylated fusion mRNA encoding both a reporter gene sequence capable of translation in said system and a heterologous sequence to be evaluated as a target for RNAi (RNAi target sequence), wherein the RNAi target sequence is positioned within either the 5' or 3' untranslated region of the fusion mRNA, and a dsRNA effector molecule having at least partially double-stranded RNA sequence, one strand of said sequence being substantially homologous to at least a portion of the RNAi target sequence.

18. The RNAi competent cell system of claim 17 wherein the RNAi target sequence is positioned within the 3' untranslated region of the fusion mRNA.

19. The RNAi competent cell system of claim 17 wherein the reporter gene sequence encodes a chemiluminescent or fluorometric reporter.

20. The RNAi competent cell system of claim 19 wherein the reporter is a green fluorescent protein (GFP).

21. The RNAi competent cell system of claim 20 wherein the reporter is EGFP.

22. The RNAi competent cell system of claim 17 wherein the fusion mRNA is encoded on an expression construct.

23. The method of claim 1 wherein the heterologous target sequence comprises two or more epitopes from a single target.

24. The RNAi competent cell system of claim 17 wherein the heterologous target sequence comprises two or more epitopes from a single target.

## Patentansprüche

1. Verfahren zum Beurteilen von durch dsRNA vermitteltem Silencing oder Inhibition einer Targetnukleotidsequenz durch ein ausgewähltes dsRNA-Effektormolekül in einem RNAi-kompetenten System, umfassend die Schritte:
a) das Einbringen in das System:
i) einer mit einem Cap versehenen und polyadenylierten Fusions-mRNA, die sowohl eine Reportergensequenz, die in dem System translationsfähig ist, als auch eine heterologe Sequenz kodiert, um als Target für RNAi beurteilt zu werden (RNAi-Targetsequenz), wobei die RNAi-Targetsequenz sich entweder innnerhalb der 5'-oder der 3'-nichttranslatierten Region der Fusions-mRNA befindet; sowie
ii) eines dsRNA-Effektormoleküls mit mindestens teilweise doppelsträngiger RNA-Sequenz, wobei ein Strang dieser Sequenz im Wesentlichen homolog zu mindestens einem Teil der RNAi-Targetsequenz ist; und
b) das Nachweisen der Gegenwart des Reportergenprodukts.

2. Verfahren gemäß Anspruch 1, wobei sich die RNAi-Targetsequenz innerhalb der 3'-nichttranslatierten Region der Fusions-mRNA befindet.

3. Verfahren gemäß einem der Ansprüche 1 oder 2, wobei die Reportergensequenz einen chemilumineszenten oder fluorometrischen Reporter kodiert.

4. Verfahren gemäß einem der Ansprüche 1 bis 3, wobei die Reportergensequenz einen fluorometrischen Reporter kodiert.

5. Verfahren gemäß Anspruch 4, wobei der fluorometrische Reporter ein grünfluoreszierendes Protein (GFP) ist.

6. Verfahren gemäß Anspruch 5, wobei der Reporter EGFP ist.

7. Verfahren gemäß einem der Ansprüche 1 oder 3 bis 6, wobei die RNAi-Targetsequenz eine Sequenz eines Pathogens, einer endogenen Sequenz, die mit Krankheit oder Pathologie in einem Wirbeltier assoziiert ist, oder ein Transgen, dessen Modulation gewünscht wird, ist.

8. Verfahren gemäß Anspruch 7, wobei das Pathogen ein Virus, Bakterium, Pilz, Nematode oder Prion ist.

9. Verfahren gemäß Anspruch 8, wobei das Virus HBV, HCV, HIV, HSV, HPV, CMV, EBV oder HTLV ist.

10. Verfahren gemäß Anspruch 7, wobei die endogene Sequenz von TNF alpha, einer Krebs-assoziierten Sequenz oder ein Wirtsgen, das für den Eintritt oder die Infektion durch ein Pathogen verantwortlich ist, ist.

11. Verfahren gemäß einem der Ansprüche 1 oder 2 bis 10, wobei das RNAi-kompetente System eine Zelle ist.

12. Verfahren gemäß Anspruch 11, wobei die Zelle eine RD-Zelle, eine Huh7-Zelle oder eine HeLa-Zelle ist.

13. Verfahren gemäß einem der Ansprüche 1 oder 2 bis 12, wobei die Fusions-mRNA in der Zelle exprimiert wird.

14. Verfahren gemäß einem der Ansprüche 1 oder 2 bis 13, wobei die Fusions-mRNA von einem Plasmid exprimiert wird.

15. Verfahren gemäß einem der Ansprüche 1 oder 2 bis 14, wobei die Fusions-mRNA und die Effektor-dsRNA beide in der Zelle exprimiert werden.

16. Verfahren gemäß einem der Ansprüche 1 oder 2 bis 15, wobei sowohl die Fusions-mRNA als auch die Effektor-dsRNA von einem oder mehr Plasmiden exprimiert werden.

17. RNAi-kompetentes Zellsystem, umfassend eine mit einem Cap versehene und polyadenylierte Fusions-mRNA, die sowohl eine Reportergensequenz, die in dem System translationsfähig ist, als auch eine heterologe Sequenz, die als Target für RNAi beurteilt werden soll (RNAi-Targetsequenz), wobei die RNAi-Targetsequenz entweder innerhalb der 5'- oder 3'-nichttranslatierten Region der Fusions-mRNA positioniert ist, umfasst, sowie ein dsRNA-Effektormolekül, das eine mindestens teilweise doppelsträngige RNA-Sequenz hat, wobei ein Strang dieser Sequenz im Wesentlichen homolog zu mindestens einem Teil der RNAi-Targetsequenz ist.

18. RNAi-kompetentes Zellsystem gemäß Anspruch 17, wobei sich die RNAi-Targetsequenz innerhalb der 3'-nichttranslatierten Region der Fusions-mRNA befindet.

19. RNAi-kompetentes Zellsystem gemäß Anspruch 17, wobei die Reportergensequenz einen chemilumineszenten oder fluorometrischen Reporter kodiert.

20. RNAi-kompetentes Zellsystem gemäß Anspruch 19, wobei der Reporter ein grünfluoreszierendes Protein (GFP) ist.

21. RNAi-kompetentes Zellsystem gemäß Anspruch 20, wobei der Reporter EGFP ist.

22. RNAi-kompetentes Zellsystem gemäß Anspruch 17, wobei die Fusions-mRNA von einem Expressionskonstrukt kodiert wird.

23. Verfahren gemäß Anspruch 1, wobei die heterologe Targetsequenz zwei oder mehr Epitope von einem einzelnen Target umfasst.

24. RNAi-kompetentes Zellsystem gemäß Anspruch 17, wobei die heterologe Targetsequenz zwei oder mehr Epitope von einem einzelnen Target umfasst.

## Revendications

1. Procédé pour évaluer le silençage ou l'inhibition à médiation par un ARNdb d'une séquence nucléotidique cible par une molécule effectrice d'ARNdb choisie dans un système ARNi-compétent, comprenant les étapes de:
a) introduire dans un tel système :
i) un ARNm de fusion coiffé et polyadénylé codant une séquence de gène rapporteur capable de traduction dans ledit système et une séquence hétérologue à évaluer comme cible pour un ARNi (séquence cible d'ARNi), où la séquence cible d'ARNi est positionnée dans la région non traduite en 5' ou 3' de l'ARNm de fusion; et
ii) une molécule effectrice d'ARNdb ayant une séquence d'ARN au moins partiellement double brin, un brin de ladite séquence étant sensiblement homologue d'au moins une partie de la séquence cible d'ARNi; et
b) détecter la présence du produit du gène rapporteur.

2. Procédé selon la revendication 1 où la séquence cible d'ARNi est positionnée dans la région non traduite en 3' de l'ARNm de fusion.

3. Procédé selon l'une quelconque des revendications 1 ou 2 où la séquence de gène rapporteur code un rapporteur chimiluminescent ou fluorimétrique.

4. Procédé selon l'une quelconque des revendications 1 ou 3 où la séquence de gène rapporteur code un rapporteur fluorimétrique.

5. Procédé selon la revendication 4 où le rapporteur fluorimétrique est une protéine fluorescente verte (GFP).

6. Procédé selon la revendication 5 où le rapporteur est EGFP.

7. Procédé selon l'une quelconque des revendications 1, ou 3-6 où la séquence cible d'ARNi est une séquence provenant d'un pathogène, une séquence endogène associée à une maladie ou pathologie chez un vertébré, ou un transgène que l'on souhaite moduler.

8. Procédé selon la revendication 7 où le pathogène est un virus, une bactérie, un champignon, un nématode ou un prion.

9. Procédé selon la revendication 8 où le virus est HBV, HCV, HIV, HSV, HPV, CMV, EBV ou HTLV.

10. Procédé selon la revendication 7 où la séquence endogène est de TNF alpha, une séquence associée au cancer ou un gène de l'hôte responsable de l'entrée de ou d'une infection par un pathogène.

11. Procédé selon l'une quelconque des revendications 1, ou 2-10 où le système ARNi-compétent est une cellule.

12. Procédé selon la revendication 11 où la cellule est une cellule RD, une cellule Huh7 ou une cellule HeLa.

13. Procédé selon l'une quelconque des revendications 1, ou 2-12 où l'ARNm de fusion est exprimé dans la cellule.

14. Procédé selon l'une quelconque des revendications 1, ou 2-13 où l'ARNm de fusion est exprimé à partir d'un plasmide.

15. Procédé selon l'une quelconque des revendications 1, ou 2-14 où l'ARNm de fusion et l'ARNdb effecteur sont exprimés l'un et l'autre dans la cellule.

16. Procédé selon l'une quelconque des revendications 1, ou 2-15 où l'ARNm de fusion et l'ARNdb effecteur sont exprimés à partir d'un ou plusieurs plasmides.

17. Système cellulaire ARNi-compétent comprenant un ARNm de fusion coiffé et polyadénylé codant une séquence de gène rapporteur capable de traduction dans ledit système et une séquence hétérologue à évaluer comme cible pour un ARNi (séquence cible d'ARNi), où la séquence cible d'ARNi est positionnée dans la région non traduite en 5' ou 3' de l'ARNm de fusion, et une molécule effectrice d'ARNdb ayant une séquence d'ARN au moins partiellement double brin, un brin de ladite séquence étant sensiblement homologue d'au moins une partie de la séquence cible d'ARNi.

18. Système cellulaire ARNi-compétent selon la revendication 17 où la séquence cible d'ARNi est positionnée dans la région non traduite en 3' de l'ARNm de fusion.

19. Système cellulaire ARNi-compétent selon la revendication 17 où la séquence de gène rapporteur code un rapporteur chimiluminescent ou fluorimétrique.

20. Système cellulaire ARNi-compétent selon la revendication 19 où le rapporteur est une protéine fluorescente verte (GFP).

21. Système cellulaire ARNi-compétent selon la revendication 20 où le rapporteur est EGFP.

22. Système cellulaire ARNi-compétent selon la revendication 17 où l'ARNm de fusion est codé sur une construction d'expression.

23. Procédé selon la revendication 1 où la séquence cible hétérologue comprend deux ou plusieurs épitopes provenant d'une seule cible.

24. Système cellulaire ARNi-compétent selon la revendication 17 où la séquence cible hétérologue comprend deux ou plusieurs épitopes provenant d'une seule cible.
